# EUROPEAN PATENT SPECIFICATION

(11) **EP 2 314 709 B1**
(45) Date of publication and mention of the grant of the patent: **11.05.2016**
(21) Application number: 09797345.7
(22) Date of filing: 15.04.2009
(51) Int. Cl.: C12P 7/66, C12P 7/22

(54) **Process for preparing reduced coenzyme Q10**
Verfahren zur Herstellung von reduziertem Coenzym Q10
Procédé de production de la coenzyme Q10 réduite

(30) Priority: 17.07.2008 CN 200810063121
(43) Date of publication of application: 27.04.2011
(73) Proprietor: Zhejiang Zhongning Business Co., Ltd., Zhejiang 310008 (CN)
(72) Inventor: Ren, Lei, Zhejiang 312500 (CN); Ren, Qiao, Zhejiang 312500 (CN)
(74) Representative: Kramer Barske Schmidtchen Patentanwälte PartG mbB
(86) International application number: PCT/CN2009/000398
(87) International publication number: WO 2010/006498

(56) References cited:
- EP-A1- 1 415 973
- EP-A1- 1 440 962
- EP-A1- 1 466 983
- WO-A1-01/52822
- WO-A1-2008/047882
- WO-A2-2011/078648
- CN-A- 1 525 951
- CN-A- 1 564 796
- CN-A- 1 608 136
- CN-A- 101 307 338
- JP-A- 61 293 391
- JP-A- 2003 113 129
- JP-A- 2003 119 127
- DATABASE WPI Week 200830 Thomson Scientific, London, GB; AN 2008-E28271 XP002666740, -& CN 101 078 019 A (SHANGHAI FANGYI BIOENGINEERING CO LTD) 28 November 2007 (2007-11-28)
- RAUCHOVÁ, H. ET AL.: "Steady-State Kinetics of Reduction of Coenzyme Q Analogs by Glycerol-3-phosphate Dehydrogenase in Brown Adipose Tissue Mitochondria", ARCHIVES OF BIOCHEMISTRY AND BIOPHYSICS, vol. 344, no. 1, August 1997 (1997-08), pages 235-241,

## Description

### TECHNICAL FIELD

The present invention relates to a method of producing reduced coenzyme Q₁₀, especially to a method of oxidized coenzyme Q₁₀ converting to produce reduced coenzyme Q₁₀.

### BACKGROUND ART

Coenzyme Q₁₀, also known as ubiquinone, is an oil-soluble orange crystal at room temperature. Its melting point is 48 °C, it has no smell and the structure is similar to Vitamin K. Q refers to the quinone chemical group, and 10 refers to the isoprenyl chemical subunits. The structure is as follows:

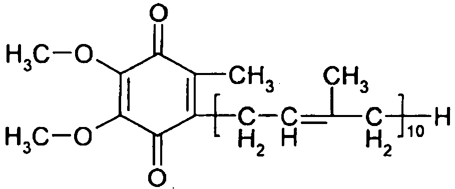

Coenzyme Q₁₀ has another form, reduced coenzyme Q₁₀, which physiological functions derive from the oxidizing and reducing characteristics of benzoquinonyl and isoprenoid chain physical characteristic. It has two main functions on human being: first, a strong effect of anti-lipid peroxidation; second, it plays an important role during the process of energy transformation from nurishments in mitochondrion. The quinone ring transfers electron and hytron in reduction breathing chain. This function is essential for life and vital for ATP. Reduced coenzyme Q₁₀ is a natural cell antioxidant and metabolism activator. Meanwhile, it protects and revives integrality of a biomembrane structure, stabilizes membrane potential and improves body's non-specific immunity. It is used as assistant treatment in heart and liver clinical curing.

Reduced coenzyme Q₁₀ has the two effects as mentioned above. Therefore, producing reduced coenzyme Q₁₀, especially natural reduced coenzyme Q₁₀, will provide a direct effect. However, the characteristic of reduced coenzyme Q₁₀ is that it is not stable; it can be easily oxidized by molecular oxygen. Thus, large-scale production of reduced coenzyme Q₁₀ is not possible. It is known that reduced coenzyme Q₁₀ can be obtained by using a reducing agent reducing coenzyme Q₁₀, such as it is disclosed in WO 01/52822 A1. There is a problem of molecule reduction in case of reduced coenzyme Q₁₀, which is reduced by a chemical reducing agent, if it is used in healthy food, beverage, cosmetic and drugs. Up to now, commercial reduced coenzyme Q₁₀ cannot be manufactured. Additionally, other problems, like how to protect and stabilize reduced coenzyme Q₁₀, have to be solved.

Futhermore, those which give rise to environmental pollution and are not safe to human health, like chemical reducing agents, protectants and additives, organic solvents, are widely used in producing reduced coenzyme Q₁₀ and exist in cosmetic, food and drugs. The harm of reducing agents and protectants is even more than the effect of reduced coenzyme Q₁₀.

JP 2003-113129 A discloses a method for producing reduced-type coenzyme Q₁₀ crystals, wherein the method comprises reducing oxidized-type coenzyme Q₁₀ by using a hyposulfite in a mixed solvent of an organic solvent and water and crystallizing the formed reduced-type coenzyme Q₁₀ from the reaction system.

DATABASE WPI, Thomson Scientific, AN 2008-E28271, Week 200830, and CN 101 078 019 A disclose a method for producing coenzyme Q₁₀ by enzyme engineering, comprising synthesizing coenzyme Q₁₀ using coenzyme Q₀ and decaisoprene, or coenzyme Q₁ and solanesol as raw materials, with polymerase, wherein the phosphorylation of coenzyme Q₀ or coenzyme Q₁ comprises dissolving coenzyme Q₀ or coenzyme Q₁ in a phosphoric acid buffer solution at 30 °C, adding phosphate to the solution with a weight of 5 % of the weight of coenzyme Q₀ and stirring for 30 minutes.

H. Rauchová et al., Archives of Biochemistry and Biophysics, Vol. 344, No. 1, 235-241, 1997, disclose a study on steady-state kinetics of reduction of coenzyme Q analogs by glycerol-3-phosphate dehydrogenase in brown adipose tissue mitochondria.

### SUMMARY OF THE INVENTION

In view of the foregoing, the present invention is targeted to offer a enzymatic conversion method of producing reduced coenzyme Q₁₀, which is very natural, green, safe and reliable.

The present invention contains following techniques:
(1) phosphorylation of oxidized coenzyme Q 10 , wherein the phosphorylation of oxidized coenzyme Q10 is carried out by dissolving the oxidized coenzyme Q10 in phosphoric acid solution, then adding phosphonolipide or phosphonolipide salt in an amount of 2 to 10 % by weight of oxidized coenzyme Q10 with 10 to 120 minutes stirring at 28 °C to 45 °C
(2) reduction of the phosphorylated oxidized coenzyme Q 10 by biological reductases,
(3) extracting reduced coenzyme Q 10 from reductases.

The reaction formula for the foregoing is as follows:

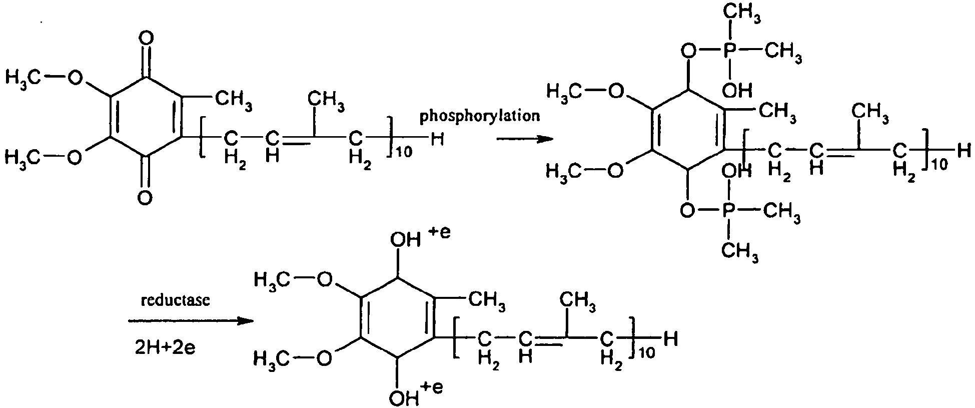

The method according to step (2), wherein the following is preferred:
(1) Choosing bacteria or endophytic fungi which contain coenzyme Q₁₀ to develop.
(2) Disrupting cells, and deposit them with ammonium sulfate of 30 % - 70 % by weight.
(3) Isolating the precipitate, dialysing the foregoing to get biologically reduced coenzyme Q₁₀.

The above phosphorylated and biologically reduced oxidized coenzyme Q₁₀ is provided by a smooth reduction and in a higher yield.

Preferably, bacteria are photosynthetic bacteria or Rhodobacter sphaeroides.

Preferably, the cells are disrupted by an Ultrasonic Cell Disruptor.

Preferably, a precipitate is washed in water of 35 °C to 45 °C for 1 to 2 hours after deposition, and it is centrifugated with a cooling centrifuge for 10 to 30 minutes at 1 °C to 3 °C and 10000 to 15000 r/min, then discard supernatant, adding phosphoric acid solution to dialysis.

More preferably, Sepharose 4B is added to stir after dialysis, and it is washed three times with phosphoric acid solution, added with sodium hydroxide to get pH 8.5, dissociating, freeze drying the liquid after filtering with cool drying machine to obtain foregoing coenzyme Q₁₀.

More preferably, the phosphoric acid solution pH range is adjusted to be 6.0 to 8.2.

Preferably, the extraction from pressure film filtering the reactants is vacuumly concentrated with adding water during the concentration, crystallization at 2 °C to 10 °C, then cooling and drying the crystals to obtain the foregoing reduced coenzyme Q₁₀.

More preferably, the pressure in the pressure film filtering is 0.1 MPa to 0.8 MPa.

By the above mentioned method of natural enzymatic conversion of oxidized coenzyme Q₁₀, it can be manufactured in a large-scale without special protective atmosphere. The foregoing obtained reduced coenzyme Q₁₀ has the following features:
(1) Stability. No reducing by dioxygen or any chemical compound. Coenzyme Q₁₀ retains its qualities in phosphoric acid solution of pH 6.0 to 8.2, and has a shelf life of over 2 years below 50 °C.
(2) Fully natural. It is compelety safe to use for food, drug and cosmetic while no reducing agent, antioxidant or protectant is used during the process.
(3) Water-solubility. It can be used for injection, since it is completely water soluble after phosphorylation.

### BRIEF DESCRIPTION OF THE DRAWING

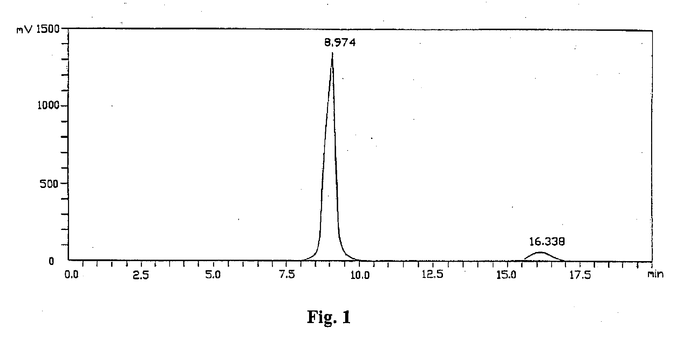

Fig. 1 is a HPLC print of reduced coenzyme Q₁₀ obtained according to the present invention.

### DETAILS OF CARRYING OUT THE INVENTION

### EXAMPLE 1

Method of using bacteria to produce biological reductase:
1. Seed selection: Rhodobacter sphaeroides, a healthy active bacteria seed of 0.5-0.9 micron width, 1.2-2.0 micron length, gammae, no capsule, single polar flagellum.
2. Preparing zymotic fluid: 5.5 g monometallic sodium orthophosphate, 1.5 g DL-malic acid, 2.0 g sodium acetate, 2.0 g sodium hydroxide, 1.0 g ammonium chloride, 0.25 g magnesium chloride, 0.05 g calcium chloride, 3.5 g glucose dissolved in 1.0 L distilled water, stirring, pH range is between 6.5-7.0.
3. Prepare 10 triangular flasks of 250 ml, pouring 100 ml inoculum into each flask after sterilization. Seal them after inoculation. Put them into incubator for 72 hours in sunlight at 32 °C, then get out.
4. Collecting inoculum, centrifugating it with centrifuge for 20 minutes, 6000 r/min, then disrupt the mycelium after centrifugation, adding 200 ml brine of 3 % by weight in still for 1 hour. Then collect supernatant, add 500 ml ammonium sulfate, wash with 40 °C water for 1.5 hour, next centrifugate with cooling centrifuge for 20 minutes at 12000 r/min at 4 °C. Then discard supernatant, add 300 ml phosphoric acid solution to dialysis, stir with 100 ml Sepharose 4B, wash three times with 200 ml phosphoric acid solution. Add sodium hydroxide to adjust to pH 8.5, dissociate, cool dry the filtered liquid with cooling drying machine to obtain 80 mg biological reductase.

### EXAMPLE 2

Method of using yeast to produce biological reductase:
1. Seed selection: Schizosaccharomyces pombe.
2. Preparing zymotic fluid: beef extract 0.3 g, peptone 1.0 g, NaCl 0.5 g, water 100 ml, adjust pH to 7.0 to 7.2, add water into beaker, weigh up beef extract, peptone and NaCl, warm them up to be melted, adjust the pH to 7.0 to 7.2, pack each with cotton tampon, then high pressure steam sterilizing to finish.
3. Preparing 10 triangular flasks of 250 ml, pouring 100 ml inoculum into each flask after sterilization. Seal them after inoculation. Put them into incubator for 72 hours in sunlight at 35 °C, then get out.
4. Collecting inoculum, centrifugating it with centrifuge for 20 minutes, 6000 r/min, then disrupt the mycelium after centrifugation, adding 200 ml brine of 3 % by weight in still for 1 hour. Then collect supernatant, add 500 ml ammonium sulfate, wash with 40 °C water for 1.5 hour, next centrifugate with cooling centrifuge for 20 minutes at 12000 r/min at 5 °C. Then discard supernatant, add 300 ml phosphoric acid solution to dialysis, stir with 100 ml Sepharose 4B, wash three times with 200 ml phosphoric acid solution. Add sodium hydroxide to adjust to pH 8.5, dissociate, cool dry the filtered liquid with cooling drying machine to obtain 80 mg biological reductase.

### EXAMPLE 3

Method of using fungus to produce biological reductase:
1. Seed selection: Endophytic
2. Preparing zymotic fluid: beef extract 0.3 g, peptone 1.0 g, NaCl 0.5 g, agar 1.5 g, water 100 ml.
3. Preparing 10 triangular flasks of 250 ml, pouring 100 ml inoculum into each flask after sterilization. Seal them after inoculation. Put them into incubator for 72 hours in sunlight at 36 °C, then get out.
4. Collecting inoculum, centrifugating it with centrifuge for 20 minutes, 6000 r/min, then disrupt the mycelium after centrifugation, adding 200 ml brine of 3 % by weight in still for 1 hour. Then collect supernatant, add 500 ml ammonium sulfate, wash with 40 °C water for 1.5 hour, next centrifugate with cooling centrifuge for 20 minutes at 12000 r/min at 2 °C. Then discard supernatant, add 300 ml phosphoric acid solution to dialysis, stir with 100 ml Sepharose 4B, wash three times with 200 ml phosphoric acid solution. Add sodium hydroxide to adjust to pH 8.5, dissociate, cool dry the filtered liquid with cooling drying machine to obtain 80 mg biological reductase.

### EXAMPLE 4

Using oxidized coenzyme Q₁₀ to react with reductase, converting into natural reduced coenzyme Q₁₀.
1. Phosphorylating the oxidized coenzyme Q₁₀: Dissolve 250 g oxidized coenzyme Q₁₀ in 1000 ml phosphoric acid solution (pH 7.4), then add 12.5 g phosphonolipide in an amount of 5 % by weight of oxidized coenzyme Q₁₀ with 10 to 120 minutes stirring under 37 °C atmosphere.
2. Prepare biological reductase according to examples 1 to 3.
3. Put the phosphorylated oxidized coenzyme Q₁₀ into reactor, add 80 g biological reductase, stir for 10 to 30 minutes at 37 °C to 60 °C atmosphere.
4. Film filtering: with a pressure of 0.5 MPa, making filtering at 30 °C, and leave the filtrate in the air for above 5 hours at 2 °C.
5. Concentrating the filtrate at 35 °C, 200 Pa vacuum atmosphere.
6. Washing the concentrate for 3 times with water, then crystallizing at 2 °C.
7. Obtaining 248.2 g crystals in the cooling drying machine. The purity reaches 99.4 %, as tested, no oxidized coenzyme Q₁₀ exists.

### EXAMPLE 5

Prepare reduced coenzyme Q₁₀ according to example 4, test it with HPLC.
Equipment: Shimazu S-10Avp; wave length: 275nm
Mobile phase: ethanol:methanol = 1:1 (v:v)
Column: octadecyl silica gel column; length: 180mm; inner diameter: 3.2 mm
   Weigh up 20 mg of reduced coenzyme Q₁₀, dissolve in ethanol to 100 ml, sample size 20 µl, using three-point external standard method to calculate assay.

Appearance time 8.974 min. Test chart see Fig. 1.

## Claims

1. A method of producing reduced coenzyme Q₁₀, including the following steps:
(1) phosphorylation of oxidized coenzyme Q₁₀, wherein the phosphorylation of oxidized coenzyme Q₁₀ is carried out by dissolving the oxidized coenzyme Q₁₀ in phosphoric acid solution, then adding phosphonolipide or phosphonolipide salt in an amount of 2 to 10 % by weight of oxidized coenzyme Q₁₀ with 10 to 120 minutes stirring at 28 °C to 45 °C;
(2) reduction of phosphorylated oxidized coenzyme Q₁₀ by biological reductases;
(3) extracting reduced coenzyme Q₁₀ from reductases.

2. The method according to claim 1, wherein the biological reductases are made by:
(1) choosing bacteria or endophytic fungi which contain coenzyme Q₁₀ to develop;
(2) disrupting cells and deposit them with ammonium sulfate of 30 % - 70 % by weight;
(3) isolating the precipitate, dialysing the foregoing to get biologically reduced coenzyme Q₁₀.

3. The method according to claim 2, wherein the bacteria are photosynthetic bacteria or Rhodobacter sphaeroides.

4. The method according to claim 2, wherein the cells are disrupted by an Ultrasonic Cell Disruptor.

5. The method according to claim 2, 3 or 4, wherein a precipitate is washed in water of 35 °C to 45 °C for 1 to 2 hours after deposition, and it is centrifugated with a cooling centrifuge for 10 to 30 minutes at 1 °C to 3 °C and 10000 to 15000 r/min, then discard supernatant, adding phosphoric acid solution to dialysis.

6. The method according to claim 5, wherein Sepharose 4B is added to stir after dialysis, and it is washed three times with phosphoric acid solution, added with sodium hydroxide to get pH 8.5, dissociating, freeze drying the liquid after filtering with cool drying machine to obtain foregoing coenzyme Q₁₀.

7. The method according to claim 1 or 2, wherein the pH range of the phosphoric acid solution is adjusted to be 6.0 to 8.2.

8. The method according to claim 1 or 2, wherein the extraction from pressure film filtering the reactants is vacuumly concentrated with adding water during the concentration; crystallization at 2 °C to 10 °C, then cooling and drying the crystals to obtain the foregoing reduced coenzyme Q₁₀.

9. The method according to claim 8, wherein the pressure in the pressure film filtering is 0.1 MPa to 0.8 MPa.

## Patentansprüche

1. Verfahren zur Herstellung von reduziertem Coenzym Q₁₀, das die folgenden Schritte umfasst:
(1) Phosphorylieren von oxidiertem Coenzym Q₁₀, wobei die Phosphorylierung von oxidiertem Coenzym Q₁₀ durch Lösen des oxidierten Coenzyms Q₁₀ in einer Phosphorsäurelösung, dann Zusetzen von Phosphonolipid oder eines Phosphonolipidsalzes in einer Menge von 2 bis 10 Gew.-% von oxidiertem Coenzym Q₁₀ mit 10 bis 120 Minuten Rühren bei 28 °C bis 45 °C durchgeführt wird,
(2) Reduzieren von phosphoryliertem oxidierten Coenzym Q₁₀ durch biologische Reduktasen,
(3) Extrahieren von reduziertem Coenzym Q₁₀ aus Reduktasen.

2. Verfahren nach Anspruch 1, bei dem die biologischen Reduktasen hergestellt werden durch:
(1) Auswählen von Bakterien oder endophytischen Pilzen, die Coenzym Q₁₀ enthalten, zur Entwicklung,
(2) Aufschließen von Zellen und Versetzen derselben mit 30 bis 70 Gew.-%igem Ammoniumsulfat,
(3) Isolieren des Niederschlags und Dialysieren desselben, so dass biologisch reduziertes Coenzym Q₁₀ erhalten wird.

3. Verfahren nach Anspruch 2, bei dem die Bakterien photosynthetische Bakterien oder Rhodobacter sphaeroides sind.

4. Verfahren nach Anspruch 2, bei dem die Zellen durch ein Ultraschall-Zellaufschlussgerät aufgeschlossen werden.

5. Verfahren nach Anspruch 2, 3 oder 4, bei dem ein Niederschlag in Wasser mit 35 °C bis 45 °C für 1 bis 2 Stunden nach dem Versetzen gewaschen und mit einer Kühlzentrifuge für 10 bis 30 Minuten bei 1 °C bis 3 °C und 10000 bis 15000 Umdrehungen/Minute zentrifugiert wird, worauf der Überstand verworfen wird und eine Phosphorsäurelösung zur Dialyse zugesetzt wird.

6. Verfahren nach Anspruch 5, bei dem nach der Dialyse Sepharose 4B zugesetzt und gerührt wird und dreimal mit einer Phosphorsäurelösung gewaschen wird, worauf Natriumhydroxid zum Erhalten eines pH-Werts von 8,5 zugesetzt wird, eine Dissoziation durchgeführt wird, die Flüssigkeit nach dem Filtrieren mit einem Kältetrocknungsgerät gefriergetrocknet wird, so dass das vorstehend genannte Coenzym Q₁₀ erhalten wird.

7. Verfahren nach Anspruch 1 oder 2, bei dem der pH-Bereich der Phosphorsäurelösung auf 6,0 bis 8,2 eingestellt wird.

8. Verfahren nach Anspruch 1 oder 2, bei dem der Extrakt von einem Druckfilmfiltern der Reaktanten vakuumkonzentriert wird, wobei während der Konzentrierung Wasser zugesetzt wird, wobei eine Kristallisation bei 2 °C bis 10 °C durchgeführt wird und dann abgekühlt wird und die Kristalle getrocknet werden, so dass das vorstehend genannte Coenzym Q₁₀ erhalten wird.

9. Verfahren nach Anspruch 8, bei dem der Druck bei dem Druckfilmfiltern 0,1 MPa bis 0,8 MPa beträgt.

## Revendications

1. Procédé de production de la coenzyme Q₁₀ réduite, comprenant les étapes suivantes:
(1) la phosphorylation de la coenzyme Q₁₀ oxydée, dans lequel la phosphorylation de la coenzyme Q₁₀ oxydée est exécutée en dissolvant la coenzyme Q₁₀ oxydée dans une solution d'acide phosphorique, en ajoutant ensuite un phosphonolipide ou un sel de phosphonolipide en une quantité de 2 % à 10 % en poids de la coenzyme Q₁₀ oxydée avec une agitation pendant 10 à 120 minutes à 28°C jusqu'à 45°C;
(2) la réduction de la coenzyme Q₁₀ oxydée phosphorylée par des réductases biologiques; et
(3) l'extraction de la coenzyme Q₁₀ réduite des réductases.

2. Procédé selon la revendication 1, dans lequel les réductases biologiques sont constituées en:
(1) choisissant des bactéries ou des champignons endophytes qui contiennent la coenzyme Q₁₀ à développer;
(2) désintégrer des cellules et les déposer avec du sulfate d'ammonium de 30 % en poids à 70 % en poids; et
(3) isoler le précipité et dialyser celui-ci pour obtenir la coenzyme Q₁₀ biologiquement réduite.

3. Procédé selon la revendication 2, dans lequel les bactéries sont des bactéries photosynthétiques ou des Rhodobacter sphaeroïdes.

4. Procédé selon la revendication 2, dans lequel les cellules sont désintégrées par un désintégrateur de cellules ultrasonique.

5. Procédé selon l'une quelconque des revendications 2, 3 ou 4, dans lequel un précipité est lavé dans une eau à 35°C à 45°C pendant 1 à 2 heure(s) après le dépôt, et il est centrifugé avec une centrifugeuse de refroidissement pendant 10 à 30 minutes à 1°C jusqu'à 3°C et à 10000 tours/minute jusqu'à 15000 tours/minute, le surnageant est écarté et une solution d'acide phosphorique est ajoutée pour la dialyse.

6. Procédé selon la revendication 5, dans lequel de la Sepharose 4B est ajoutée pour l'agitation après la dialyse, et est lavée trois fois avec une solution d'acide phosphorique, de l'hydroxyde de sodium est ajouté pour parvenir à pH 8,5, le liquide est alors dissocié et cryo-séché après une filtration à l'aide d'une machine de séchage à froid pour obtenir la coenzyme Q₁₀ ci-dessus.

7. Procédé selon la revendication 1 ou 2, dans lequel la plage de pH de la solution d'acide phosphorique est ajustée pour se situer entre 6,0 et 8,2.

8. Procédé selon la revendication 1 ou 2, dans lequel l'extraction par filtration par membrane sous pression des réactifs est concentrée sous vide en ajoutant de l'eau pendant la concentration; une cristallisation est ensuite effectuée à 2°C jusqu'à 10°C; les cristaux étant ensuite refroidis et séchés pour obtenir la coenzyme Q₁₀ réduite ci-dessus.

9. Procédé selon la revendication 8, dans lequel la pression dans la filtration par membrane sous pression est comprise entre 0,1 MPa et 0,8 MPa.
